# EUROPEAN PATENT SPECIFICATION

(11) **EP 1 456 191 B1**
(45) Date of publication and mention of the grant of the patent: **05.07.2006**
(21) Application number: 02785688.9
(22) Date of filing: 18.12.2002
(51) Int. Cl.: C07D 301/32, C07D 301/10

(54) **PROCESS FOR PREPARING ETHYLENE OXIDE**
VERFAHREN ZUR HERSTELLUNG VON ETHYLENOXID
PROCEDE DE PREPARATION D'OXYDE D'ETHYLENE

(30) Priority: 21.12.2001 FR 0116614
(43) Date of publication of application: 15.09.2004
(73) Proprietor: INNOVENE EUROPE LIMITED, Staines Middlesex, TW18 1DT (GB); BP Lavéra SNC, 95866 Cergy Pontoise Cedex (FR)
(72) Inventor: ANDREIS, Gilbert, F-13120 Gardanne (FR); JOSTEN, Georges, F-34090 Montpellier (FR); MENE, Didier, F-13620 Carry le Rouet (FR)
(74) Representative: Preece, Michael
(86) International application number: PCT/GB2002/005769
(87) International publication number: WO 2003/055869

(56) References cited:
- EP-A- 0 322 323
- WO-A-96/16953
- WO-A-98/33785
- US-A- 3 418 338
- US-A- 4 134 797

## Description

The present invention relates to the recovery of an ethylene oxide essentially free of water and of aldehyde impurities, formaldehyde and acetaldehyde, from a mixture of water and of impure ethylene oxide obtained in particular during the manufacture of ethylene oxide.

The impure ethylene oxide comprising the aldehyde impurities in the form of a mixture with water generally results from the manufacture of ethylene oxide by gas-phase catalytic oxidation of ethylene by molecular oxygen. The catalytic oxidation can be carried out by virtue of a silver-comprising catalyst at a high temperature, for example from 100 to 500°C, under an absolute pressure greater than atmospheric pressure, for example from 0.5 to 5 MPa, in particular according to the process disclosed in United States Patent US 2 775 510 or in International Patent Application WO 96/33182.

A gaseous reaction mixture comprising ethylene oxide, the aldehyde impurities, formaldehyde and acetaldehyde, unreacted reactants, such as ethylene and oxygen, and optionally inert gases, such as nitrogen, argon, methane, ethane and carbon dioxide, results from the catalytic oxidation. The recovery of the ethylene oxide from this gaseous reaction mixture is usually carried out in several stages.

A first stage generally comprises absorption (or washing) with water, generally consisting in bringing the gaseous reaction mixture into contact with water or an essentially aqueous stream, in order to form a very dilute aqueous solution of ethylene oxide and thus to separate the ethylene oxide from the other gaseous constituents, in particular unreacted reactants and inert gases. The gaseous constituents thus separated generally constitute a gas stream for recycling which is returned to the catalytic oxidation stage, optionally after a stage of decarbonation which consists in removing the carbon dioxide gas. The very dilute aqueous solution of ethylene oxide generally comprises from 2 to 3 % by weight of ethylene oxide, the aldehyde impurities and some dissolved residual gases.

A second stage can subsequently comprise desorption (or extraction) with steam, which generally consists in passing steam, generally countercurrentwise to the very dilute aqueous solution of ethylene oxide, and in extracting from this solution, in particular the ethylene oxide. Simultaneously, an aqueous solution highly depleted in ethylene oxide is separated and, preferably, is recycled in the water or in the aqueous stream employed in the preceding stage (the absorption stage). The ethylene oxide extracted from the desorption stage generally appears in the form of a mixture of water and of impure ethylene oxide comprising the aldehyde impurities, formaldehyde and acetaldehyde. Generally, this mixture is in a ratio by weight of ethylene oxide to water of 0.1/1 to 4/1, preferably of 0.2/1 to 3/1, in particular of 0.4/1 to 1.6/1. In view of the desorption conditions, the mixture of water and of impure ethylene oxide thus extracted generally appears in the form of a gas mixture.

The stages which subsequently follow for separating the ethylene oxide from the water and the aldehyde impurities differ from one process to another and generally exhibit failings and disadvantages. Thus, for example, United States Patent US 3 904 656 discloses a stage of reabsorption by water of the ethylene oxide present in the mixture of water and impure ethylene oxide resulting from the desorption stage. A large part of the dissolved residual gases is not reabsorbed in the water and is easily separated in the form of a gas stream. Furthermore, the reabsorption results in a fresh dilution of the ethylene oxide in water, thus forming a dilute aqueous solution of ethylene oxide (for example, of 5 to 15 % by weight of ethylene oxide) comprising the aldehyde impurities. The following stages can successively comprise a "refining" stage, a stage of extraction of carbon dioxide gas and a final stage of purification of the ethylene oxide, so as to remove in particular acetaldehyde. However, the ethylene oxide thus treated still comprises substantial amounts of aldehyde impurities and in particular of formaldehyde, for example more than 10 parts by weight per million (ppm) of formaldehyde. Furthermore, the "refining" stage and the stage of extraction of carbon dioxide gas lead to not insignificant losses of ethylene oxide, so that the yield of purified ethylene oxide is relatively low. Finally, the dilution of the ethylene oxide by fresh amounts of water during the reabsorption stage inevitably leads subsequently to a considerable expenditure of energy in separating the ethylene oxide from this added water.

United States Patents US 4 134 797 and US 5 529 667 also disclose a stage of reabsorption of the ethylene oxide by water and a stage of extraction of the carbon dioxide gas, as described above, and additionally provide a final stage of purification intended to reduce the content of aldehyde impurities in the ethylene oxide. This final stage is carried out by addition of steam as entrainment fluid with the mixture of water and of impure ethylene oxide in a distillation column. Pure ethylene oxide substantially free of aldehyde impurities is withdrawn by a first side stream from the distillation column, while impure ethylene oxide (rich in formaldehyde and/or acetaldehyde) is withdrawn simultaneously by the top of the distillation column and by a second side stream. In particular, the stream withdrawn by the top of the distillation column is a stream of impure ethylene oxide (about 99.5 % by weight or more of ethylene oxide) rich in formaldehyde (approximately from 50 to 500 ppm of formaldehyde), the stream withdrawn by the first side stream is a stream of pure ethylene oxide substantially free of aldehyde impurities (approximately from 10 to 350 ppm of aldehyde impurities and generally with less than 20 or even less than 10 or 5 ppm of formaldehyde), and the stream withdrawn by the second side stream is a stream of impure ethylene oxide (about 88 or 95 % by weight or more of ethylene oxide) rich in acetaldehyde (approximately from 0.1 to 10 %, or from 0.2 to 2 % by weight of acetaldehyde). However, the stream of pure ethylene oxide is only obtained at the cost of a very complex process which leaves approximately from 25 to 40 % of the ethylene oxide in the unpurified form, without counting the losses of ethylene oxide which occur during the stages of reabsorption and of decarbonation and the disadvantages described above related to these two stages.

European Patent Application EP 0 322 323 discloses a process for separating ethylene oxide from aldehyde impurities, comprising first of all a stage of reabsorption with water of the ethylene oxide, as described in the last two United States patents, or else a "gradual condensation" stage, as disclosed in European Patent Application EP 0 139 601, carried out in particular in two or three heat exchangers arranged in series, and subsequently provides for completion by a final stage of purification of a liquid stream of water and of impure ethylene oxide in a relatively high proportion of water (for example, from 1.2 to 5.2 % by weight of water). However, the implementation of the reabsorption or gradual condensation stage leads to relatively high losses of ethylene oxide, in particular because of the aqueous solutions or condensates withdrawn, so that the yield of purified ethylene oxide is relatively low. Furthermore, the ethylene oxide thus treated exhibits a content of aldehyde impurities, formaldehyde and acetaldehyde, which still remains relatively high, for example from 15 to more than 20 ppm.

United States Patent US 3 418 338 discloses an extractive distillation of a 0.5 % by weight solution of ethylene oxide comprising only formaldehyde.

International Patent Application WO 98/33785 discloses a distillation process for separating ethylene oxide from formaldehyde only, and not from the aldehyde impurities, i.e. formaldehyde and acetaldehyde.

The aim of the present invention is to correct the failings and to avoid the disadvantages of the processes described above. In particular, it provides for the provision of a purified ethylene oxide having in particular extremely low contents of the aldehyde impurities, formaldehyde and acetaldehyde, by a process which makes it possible to recover the purified ethylene oxide with a much higher yield than the known processes. The process provided is relatively simplified and the capital and production costs are particularly low. The process according to the invention is particularly well suited to treating the mixtures of impure ethylene oxide and of water resulting from the manufacture of ethylene oxide by the catalytic oxidation route.

The present invention relates first of all to a process for treating a mixture of impure ethylene oxide and of water in a ratio by weight of ethylene oxide to water of 0.1/1 to 4/1, preferably of 0.2/1 to 3/1, in particular of 0.4/1 to 1.6/1, comprising aldehyde impurities, formaldehyde and acetaldehyde, in order to obtain a purified ethylene oxide essentially free of water and of the aldehyde impurities, which process is characterized in that:
(1) the mixture is conveyed to a first fractionation region Z1, while a liquid stream L1 comprising at least 98 %, preferably at least 99 %, by weight of water and a portion of the aldehyde impurities is withdrawn at the bottom of Z1, and a gas stream G1 comprising more than 99 %, preferably more than 99.5 %, by weight of ethylene oxide with residual amounts of water and of the aldehyde impurities is withdrawn at the top of Z1, and
(2) the gas stream G1 is conveyed to a second fractionation region Z2, while a liquid stream L2 comprising a mixture of ethylene oxide, of water and of the aldehyde impurity(ies) is withdrawn at the bottom of Z2, a liquid stream L3 is withdrawn as a side stream from the region Z2 and is conveyed to the region Z1, and a gas stream G2 comprising the purified ethylene oxide essentially free of water and of the aldehyde impurities is withdrawn at the top of Z2.

The treatment process of the invention is particularly suited to a process for the manufacture of a purified ethylene oxide essentially free of water and of the aldehyde impurities, formaldehyde and acetaldehyde, which process comprises:
(a) synthesis of ethylene oxide in a reaction region by a gas-phase catalytic reaction between ethylene and molecular oxygen, so as to form a gaseous reaction mixture comprising ethylene oxide, the aldehyde impurities, unreacted reactants and possibly inert gases,
(b) absorption with water by bringing the gaseous reaction mixture obtained in (a) into contact with an essentially aqueous stream, so as to form a dilute aqueous solution of impure ethylene oxide comprising the aldehyde impurities, after separation of a gas stream for recycling essentially comprising the unreacted reactants and possibly the inert gases, which gas stream is at least partially returned, directly or indirectly, to the reaction region, and
(c) desorption of the ethylene oxide by entrainment of the dilute aqueous solution obtained in (b) with steam in a column, so as to form, at the column bottom, an aqueous stream essentially free of ethylene oxide and, at the top, a mixture of impure ethylene oxide and of water in a ratio by weight of ethylene oxide to water of 0.1/1 to 4/1, preferably of 0.2/1 to 3/1, in particular of 0.4/1 to 1.6/1, comprising the aldehyde impurities,
and is characterized in that the mixture of impure ethylene oxide and of water obtained in (c) is subjected to the treatment described above or to any one of the alternative forms of this treatment described subsequently.

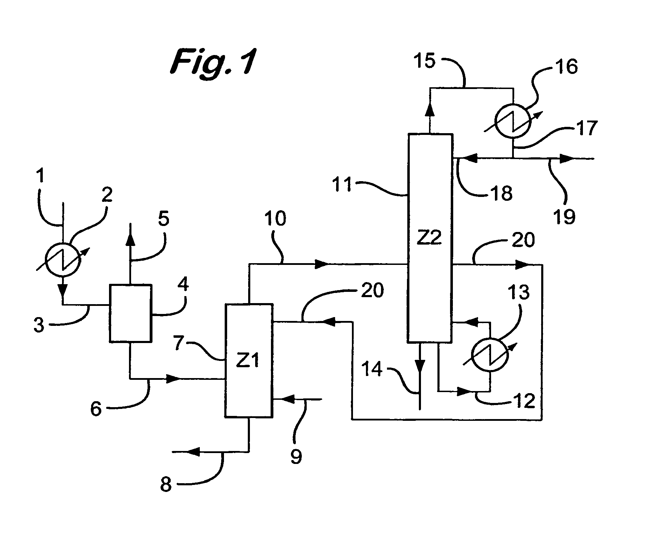

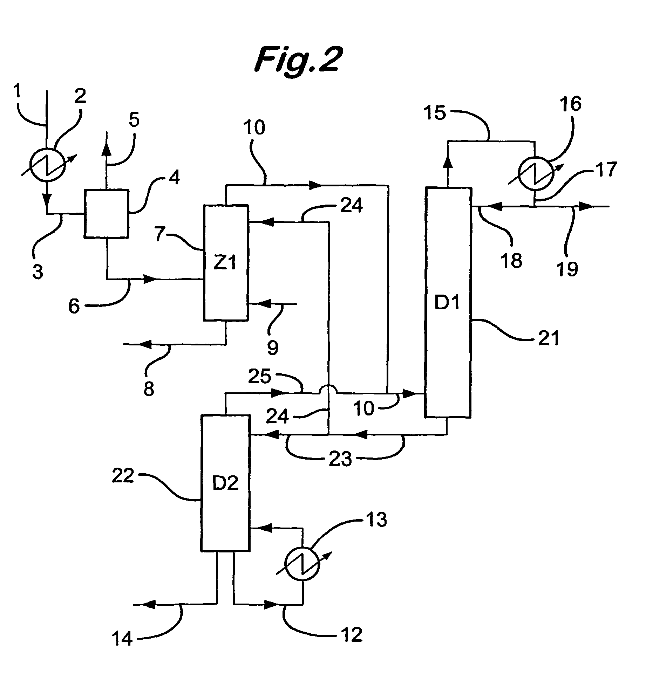

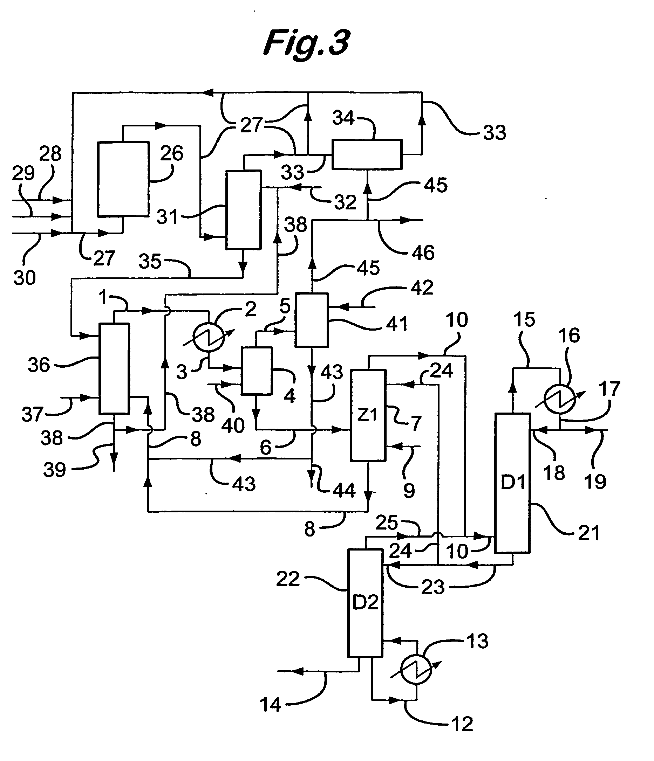

Figures 1 and 2 diagrammatically represent, by way of illustration, the process for the treatment and recovery of ethylene oxide according to the invention and a preferred alternative form of this process. Figure 3 diagrammatically represents, by way of illustration, a process for the manufacture of ethylene oxide comprising the treatment and the recovery of ethylene oxide according to the invention.

The expression "aldehyde impurities" used here and subsequently is intended to denote formaldehyde, acetaldehyde and one of their mixtures. The parts or percentages by weight of aldehyde impurities, formaldehyde and acetaldehyde, are expressed by weight of acetaldehyde in the present description, unless otherwise indicated.

Typically, the mixture of impure ethylene oxide and water which can be obtained in (c) in the process for the manufacture of ethylene oxide, or which is conveyed to the region Z1 of the treatment process according to the invention, can have a content by weight of the aldehyde impurities approximately of 50 to 2 000 parts by million by weight (ppm), preferably of 50 to 1 000 ppm, in particular of 50 to 500 ppm, with generally a molar ratio of acetaldehyde to formaldehyde of 0.5/1 to 5/1, preferably of 1/1 to 3/1 plus. In addition, the mixture can also comprise up to approximately 500 ppm, preferably up to 250 ppm, of carbon dioxide gas (with respect to the ethylene oxide present in the mixture).

In the process for the manufacture of ethylene oxide as described above, the gas stream for recycling obtained in (b) can advantageously be subjected to decarbonation before it is returned to the reaction region. The decarbonation can be carried out by any process which makes it possible to at least partially remove the carbon dioxide gas by bringing the gas stream for recycling into contact with an absorbent of the carbon dioxide gas, in particular an alkaline compound, as is described in more detail subsequently. The decarbonation of the gas stream for recycling can be a particularly effective means for reducing, controlling and regulating the content of carbon dioxide gas in the gaseous reaction mixture and also in the ethylene oxide obtained by the treatment according to the invention.

The desorption conditions in (c) of the process for the manufacture of ethylene oxide can generally be chosen so that the mixture of impure ethylene oxide and of water is obtained in the form of a gas mixture. Thus, in this case, the mixture of impure ethylene oxide and of water, prior to sending it to the region Z1 of the treatment according to the invention, can advantageously be condensed, so as to form an aqueous solution of impure ethylene oxide in proportions of water, ethylene oxide and the aldehyde impurities which are identical or essentially identical to those in the said mixture. After this condensation, but before the treatment according to the invention, it is also possible to separate, from this final solution, the possible final traces of very volatile compounds, such as residual amounts of carbon dioxide gas and possibly of one or more inert gases (such as mentioned above), by entrainment, for example with steam. This final separation can advantageously be carried out without substantially modifying the composition of the solution in ethylene oxide, water and the aldehyde impurities and preferably without substantially affecting the yield of purified ethylene oxide of the process. Such a separation can be carried out in particular under conditions as described subsequently in Figure 3. The advantage of this final separation, combined with the decarbonation stage described above, is to be able to treat the mixture of water and of impure ethylene oxide freed from substantial traces of carbon dioxide gas and consequently to use treatment equipment, such as distillation columns, condensers and reboilers, made of an ordinary steel and no longer of a special steel or of stainless steel. Thus, the aqueous solution of impure ethylene oxide, after this optional final separation, can be conveyed to the fractionation region Z1, for example at a temperature of 20 to 60°C, preferably of 30 to 50°C.

The fractionation region Z1 can comprise at least one distillation column having e.g. from 10 to 30, preferably from 15 to 25, theoretical plates. The distillation column can be a packed column or a plate column. The region Z1 can be fed with a mixture of impure ethylene oxide and of water (or preferably with an aqueous solution of impure ethylene oxide) at a level situated between a tenth and a half, preferably between an eighth and a third, of the number of theoretical plates of Z1, counting from the bottom of Z1, e.g. at a level from the 3^{rd} to the 7^{th}, preferably from the 5^{th} to the 7^{th} theoretical plates counting from the bottom of Z1. If the region Z1 comprises a packed column, the packing may have a specific substance exchange surface in the range from 200 to 500, preferably from 350 to 500 m²/m³.

The fractionation region Z1 can advantageously operate with a bottom temperature of 100 to 160°C, preferably of 120 to 150°C, in particular of 130 to 150°C and a top temperature of 30 to 60°C, preferably of 40 to 50°C, under an absolute pressure of 0.1 to 1 MPa, preferably of 0.15 to 0.5 MPa.

Heat is generally supplied to the region Z1 by virtue of a reboiler positioned at the bottom of Z1 or by virtue of the introduction of steam into the bottom of Z1. The choice of a relatively high bottom temperature in Z1 compels preference to be given to the introduction of steam instead of the use of a reboiler.

The fractionation region Z1 operates according to the invention so that a liquid stream L1 comprising at least 98 %, preferably at least 99 %, by weight of water is withdrawn at the bottom and so that a gas stream G1 comprising more than 99 %, preferably more than 99.5 %, by weight of ethylene oxide is withdrawn at the top. In particular, the operating conditions of Z1 can be such that the liquid stream L1 can comprise less than 0.5 %, preferably less than 0.2 %, by weight of ethylene oxide. It has been observed, in a particularly advantageous way, that the liquid stream L1 can comprise from 60 to 99 %, preferably from 75 to 98 %, by weight of the amount of formaldehyde present in the mixture of impure ethylene oxide and of water conveyed to the region Z1. It is conceivable that, in the process of the invention, the great majority, if not all, of the formaldehyde is separated from the ethylene oxide in the region Z1, by virtue in particular of the specific fractionation conditions applied in Z1, thus making it possible to achieve the desired compositions of the streams L1 and G1, as mentioned above: in particular, a composition of the liquid stream L1 at the bottom which is extremely rich in water and a composition of the gas stream G1 at the top which is extremely rich in ethylene oxide. Consequently, it is conceivable that the success of the process of the invention probably arises from the fact that the majority, if not all, of the formaldehyde can be separated from the ethylene oxide in the region Z1. The formaldehyde will be separated from the ethylene oxide with much more difficulty in the region Z2, where the residual amount of water and of acetaldehyde in particular can still be efficiently separated. It is also conceivable, without being able to show it with certainty, that such results can be obtained in part by virtue of the choice of a bottom temperature for Z1 which is particularly high and which might promote the preferential removal, with the liquid stream L1, of the formaldehyde in a relatively unstable hydrated form or in the form of a product from the hydration of formaldehyde.

The fractionation region Z1 can operate with the streams G1 and L1, which are withdrawn from the region Z1, in a ratio by weight of G1 to L1 of 1/1 to 5/1, preferably of 1.5/1 to 4/1, in particular of 2/1 to 3/1.

According to the invention, the gas stream G1 feeds the fractionation region Z2, which can comprise at least one distillation column having from 50 to 120, preferably from 80 to 110, in particular from 85 to 105 theoretical plates. The distillation column can be a packed column or a plate column. If the region Z2 comprises a packed column, the packing may have a specific substance exchange surface in the range from 200 to 500, preferably from 350 to 500 m²/m³.The feeding of the region Z2 with the gas stream G1 can advantageously be carried out at a level situated between a third and a half, preferably between a quarter and a half of the number of theoretical plates of Z2, counting from the bottom of Z2.

The fractionation region Z2 can advantageously operate with a bottom temperature of 35 to 65°C, preferably of 40 to 60°C, and a top temperature of 30 to 50°C, preferably 35 to 45°C, under an absolute pressure which is different from or, preferably, substantially identical to that existing in the region Z1, in particular an absolute pressure of 0.1 to 1 MPa, preferably of 0.15 to 0.5 MPa. It can be of a particular interest that the top temperature is so low, since the purified ethylene oxide is withdrawn at the top of the region Z2 and then can be easily condensed in a condensate form at a particularly low temperature, e.g. from 15 to 30°C.

Heat is generally supplied to the region Z2 by virtue of the introduction of steam into the bottom of Z2 or, preferably, by virtue of a reboiler positioned at the bottom of Z2, due in particular to the low temperature applied in the bottom of Z2.

A particularly advantageous alternative form of the operation of the fractionation region Z2 can be that the region Z2 operates at reflux. Thus, the gas stream G2 withdrawn at the top of Z2 can be advantageously condensed and preferably cooled, so as to form a condensate at a temperature, for example, of 15 to 30°C. A portion of the resulting condensate is returned as reflux to the top of the region Z2 and the additional non-refluxed portion is recovered in the form of a liquid stream composed of the purified ethylene oxide essentially free of water and of the aldehyde impurities such as resulting from the treatment according to the invention. The reflux ratio, expressed by a ratio by weight of the refluxed part to the additional non-refluxed part, can advantageously be from 1/1 to 10/1, preferably from 1.5/1 to 5/1, in particular from 2.5/1 to 4/1. An excessively low reflux ratio can affect the quality of the ethylene oxide withdrawn and recovered at the top of Z2 and can in particular increase the content of the aldehyde impurities and in particular of acetaldehyde in the ethylene oxide.

The fractionation region Z2 operates according to the invention so that a liquid stream L2, comprising a mixture of ethylene oxide, of water and of the aldehyde impurity(ies), is withdrawn at the bottom and so that a gas stream G2, comprising the purified ethylene oxide essentially free of water and of the aldehyde impurities, is withdrawn at the top.

The fractionation region Z2 can operate with the streams G2 and L2, which are withdrawn from the region Z2, in a ratio by weight of G2 to L2 of 30/1 to 150/1, preferably of 40/1 to 130/1, in particular of 60/1 to 100/1, especially of 65/1 to 85/1.

In particular, it is possible to use fractionation conditions such that, per 100 parts by weight of liquid stream L2, from 80 to 95 parts by weight of ethylene oxide, from 1 to 5 parts by weight of water and from 1 to 5 parts by weight of the aldehyde impurity(ies) are recorded. It is particularly interesting to note that the liquid stream L2 generally comprises, as the aldehyde impurity(ies), essentially acetaldehyde and that consequently the region Z2 serves in particular to separate the ethylene oxide from the acetaldehyde and the final traces of water. Furthermore, per 100 parts by weight of the liquid stream L2, the presence of 1 to 10 parts by weight of ethylene glycol can also be recorded.

The effectiveness of the treatment according to the invention is apparent in the composition of the purified ethylene oxide essentially free of water and of the aldehyde impurities, formaldehyde and acetaldehyde, resulting from the gas stream G2 withdrawn at the top of Z2 or in particular from the condensate formed after condensation of G2: the ethylene oxide generally comprises from 1 to 50 ppm, preferably from 2 to 10 ppm, in particular from 3 to 8 ppm, of water and from 1 to less than 15 ppm, preferably from 2 to 12 ppm, in particular from 3 to 10 ppm, of aldehyde impurities. It is particularly interesting to note that the purified ethylene oxide treated according to the invention generally comprises less than 5 ppm, preferably less than 4 ppm, in particular less than 3 ppm, of formaldehyde. Furthermore, it may also be noted that the purified ethylene oxide obtained according to the invention can comprise from 3 to 15 ppm, preferably from 3 to 10 ppm, in particular from 3 to 6 ppm, of carbon dioxide gas.

Another particularly advantageous form of the treatment process according to the invention is that a liquid stream L3 is withdrawn as a side stream from the region Z2 and is conveyed to the region Z1. Sending a liquid stream L3 to Z1 can be equivalent to operating the region Z1 under conditions similar to reflux. The liquid stream L3 can in particular be removed from Z2 at the same level as or below the point for feeding Z2 with the gas stream G1, preferably at a level situated between a third and a half, preferably between a quarter and a half of the number of theoretical plates, counting from the bottom of Z2. It has been found to be particularly advantageous to convey the liquid stream L3 to a point situated in the upper half of the region Z1, preferably at the top of Z1. Another advantageous condition intended in particular to improve the operation of the region Z1 can result from the fact that the streams G1 and L3 are employed in a ratio by weight of G1 to L3 of 0.5/1 to 5/1, preferably of 1/1 to 3/1, in particular of 1.5/1 to 2.5/1. Under these conditions, it has been observed that optimum fractionation is obtained in Z1, in particular for reducing the aldehyde impurities, and in particular formaldehyde, in the gas stream G1 exiting at the top of Z1. Finally, the streams G2 and L3 can advantageously be withdrawn from the region Z2 in a ratio by weight of G2 to L3 of 1/1 to 20/1, preferably of 2/1 to 10/1, in particular of 2.5/1 to 4/1.

It has been noticed that, by simultaneously varying the reflux ratio of the region Z2 and the ratio by weight of the streams G1 and L3, it is possible to considerably reduce the content of the aldehyde impurities, formaldehyde and acetaldehyde, in the ethylene oxide treated according to the invention, while maintaining a yield of purified ethylene oxide at a particularly high level.

Another alternative form of the treatment process according to the invention can be that the fractionation region Z2, comprises two distillation columns arranged in series, an upper column D1 and a lower column D2, so that the gas stream G1 emerges in the lower half of D1, preferably in the bottom of D1, so that the gas stream G2 exits via the top of D1, so that the bottom of D1 communicates via a liquid transfer stream TL with the top of D2, so that the top of D2 communicates via a gas transfer stream TG with the bottom of D1, and so that the liquid stream L2 exits via the bottom of D2. The distillation columns D1 and D2 can be plate columns and/or packed columns, preferably with the above-mentioned type of packing.

In this novel alternative form, the gas transfer stream TG can emerge in the gas stream G1, before being introduced into the lower half of D1 or in particular into the bottom of D1. Furthermore, the liquid stream L3 described above can be removed from the liquid transfer stream TL.

The treatment process of the invention is particularly advantageous when it is implemented continuously.

The present invention also relates to a process for the manufacture of a purified ethylene oxide essentially free of water and of the aldehyde impurities, formaldehyde and acetaldehyde, as described above, comprising in particular a stage (a) of synthesis of ethylene oxide, a stage (b) of absorption with water, a stage (c) of desorption, and a final stage which consists in treating the mixture of impure ethylene oxide and of water obtained in (c) by the process according to the invention.

The stage (a) of synthesis is known per se and comprises a gas-phase catalytic oxidation reaction of ethylene with molecular oxygen, in particular with atmospheric oxygen, in a reaction region. The reaction can be carried out in the presence of a silver-comprising catalyst and of one or more inert gases, such as nitrogen, argon, methane and ethane, at a temperature of 100 to 500°C, preferably of 150 to 300°C, under an absolute pressure of greater than atmospheric pressure, for example from 0.5 to 5 MPa, preferably from 1 to 3 MPa. The gaseous reaction mixture resulting from stage (a) generally comprises the ethylene oxide formed (from 1 to 3 mol%), the unreacted reactants, in particular oxygen (from 3 to 6 mol%) and ethylene (from 5 to 30 mol%), carbon dioxide (from 3 to 12 mol%), one or more inert gases such as those mentioned above (up to 80 mol%), and aldehyde impurities, formaldehyde and acetaldehyde.

The stage (b) of absorption with water is also known per se and can be in particular that described above. It can comprise contacting, generally countercurrentwise, the gaseous reaction mixture obtained in (a), cooled beforehand to a temperature of 50 to 100°C, with water or an essentially aqueous stream in an absorption column, at a temperature of 10 to 50°C, preferably of 20 to 40°C, under an absolute pressure of 0.5 to 5 MPa, preferably of 1 to 3 MPa. The gaseous constituents separated in this stage generally constitute a gas stream for recycling which is at least partially returned, directly or indirectly, to the reaction region of stage (a). The gas stream for recycling generally comprises the unreacted reactants, ethylene and oxygen, one or more inert gases (such as those mentioned above), and carbon dioxide. At least a portion of the gas stream for recycling is preferably subjected to a stage of decarbonation, before being returned to stage (a). The stage of decarbonation generally consists in bringing the gas stream for recycling into contact with an absorbent of carbon dioxide gas, in particular an alkaline compound, in particular an alkali metal carbonate, such as potassium carbonate, or alternatively in particular an alkanolamine, such as diisopropanolamine, an alkoylalkanolamine, or an alkali metal salt of an amino acid. The decarbonation stage is disclosed, for example, in United States Patents US 3 665 678, US 3 867 113 and US 4 184 855, in European Patent Application EP 0 583 828, and in French Patent Application FR 2 237 896. A particularly effective decarbonation process, by reversible chemical absorption in a hot aqueous potassium carbonate solution, a process known under the name of the "Benfield process" and provided by UOP, is described in "SRI International, Process Economics Program Report 2F, Ethylene Oxide and Ethylene Glycol" (January 1997), 5-10 and 5-11. The decarbonation of the gas stream for recycling makes it possible to reduce, to control and to regulate the content of carbon dioxide in the gaseous reaction mixture resulting from stage (a), as well as in the dilute aqueous solution resulting from stage (b) and finally in the mixture of impure ethylene oxide and of water resulting from stage (c), so that the final mixture can have an extremely low content of carbon dioxide gas at the point when it is to be subjected to the treatment according to the invention.

The stage (c) of desorption is also known per se and can be that described above. It generally comprises entrainment with steam of the dilute aqueous solution obtained in (b) and heated beforehand to a temperature of 70 to 110°C. The entrainment with steam can be carried out in a desorption column at a temperature of 80 to 140°C, preferably of 90 to 130°C, under an absolute pressure of 0.1 to 1 MPa, preferably of 0.1 to 0.5 MPa. An aqueous stream essentially freed from ethylene oxide, at a temperature of 90 to 130°C, is generally withdrawn at the column bottom, whereas the mixture of impure ethylene oxide and of water, ready to be subjected to the treatment according to the invention, exits at the top.

The process for the manufacture of ethylene oxide as described above is generally carried out continuously, so that it becomes advantageous also to carry out the treatment process of the invention continuously.

Figure 1 diagrammatically represents, by way of illustration, the treatment process according to the invention. The mixture of impure ethylene oxide and of water, in a ratio by weight of ethylene oxide to water of 0.1/1 to 4/1, preferably of 0.2/1 to 3/1, in particular of 0.4/1 to 1.6/1, comprising the aldehyde impurities, formaldehyde and acetaldehyde, exists in the form of a gas mixture and feeds, via a line (1), a condenser (2), so as to form an aqueous solution of impure ethylene oxide comprising the aldehyde impurities. The latter aqueous solution exits from the condenser via a line (3) and is introduced into a receiver (4) at a temperature, for example, of 20 to 60°C, under an absolute pressure of 0.1 to 0.5 MPa. Traces of very volatile compounds, such as residual amounts of carbon dioxide gas and possibly of one or more inert gases, such as those mentioned above, can optionally be volatilized and separated from the aqueous solution present in the receiver (4) and can finally be discharged via a line (5). The aqueous solution of impure ethylene oxide, free of these possible very volatile compounds, exits from the receiver (4) via a line (6) and is introduced into a fractionation region Z1, as described above, composed of a distillation column (7). A liquid stream L1 is withdrawn at the column bottom via a line (8) and comprises, according to the invention, at least 98 %, preferably at least 99 %, by weight of water and a portion of the aldehyde impurities, in particular from 60 to 98 %, preferably from 75 to 95 %, of the amount of formaldehyde present in the mixture conveyed to the region Z1. A portion at least of the liquid stream L1 can be recovered and used in the stage (c) of desorption of the ethylene oxide described above. In the lower part of the column (7), in particular at the column bottom, heat is supplied via a steam line (9). A gas stream G1, comprising, according to the invention, more than 99 %, preferably more than 99.5 %, by weight of ethylene oxide, with residual amounts of water and of the aldehyde impurities, exits at the top of the column (7) via a line (10).

The gas stream G1 feeds, via the line (10), a fractionation region Z2 as described above and composed of a distillation column (11). The column (11) is equipped at its base with a reboiler comprising a loop (12) equipped with a heat exchanger (13). A liquid stream L2, comprising a mixture of ethylene oxide, of water and of aldehyde impurity(ies) and which, per 100 parts by weight of L2, can comprise in particular from 80 to 95 parts by weight of ethylene oxide, from 1 to 5 parts by weight of water and from 1 to 5 parts by weight of the aldehyde impurity(ies), in particular of acetaldehyde, is withdrawn at the column bottom via a line (14). At least a portion of the liquid stream L2 can be recovered and conveyed to a unit for the manufacture of ethylene glycol (not represented in Figure 1). A gas stream G2 exits at the top of the column (11) via a line (15) and comprises the purified ethylene oxide essentially free of water and of the aldehyde impurities, in particular a stream of ethylene oxide comprising from 1 to 50 ppm, preferably from 2 to 10 ppm, in particular from 3 to 8 ppm, of water and from 1 to less than 15 ppm, preferably from 2 to 12 ppm, in particular from 3 to 10 ppm, of the aldehyde impurities, formaldehyde and acetaldehyde. The gas stream G2 is condensed and preferably cooled via a condenser (16), so as to form a condensate which exits from the condenser via a line (17). A portion of the condensate is returned as reflux via a line (18) to the top of the column (11) and the additional non-refluxed portion is recovered in a line (19) in the form of a liquid stream of the purified oxide essentially free of water and of the aldehyde impurities as resulting from the process according to the invention. A liquid stream L3, as described above, is withdrawn as a side stream from the column (11) via a line (20) at a level substantially identical to the level where the line (10) for feeding with the gas stream G1 emerges and is conveyed to the upper part of the column (7), in particular to the top of this column.

Figure 2 diagrammatically represents, by way of illustration, an alternative form of the treatment process according to the invention, identical to the process represented in Figure 1, except for the fact that the fractionation region Z2, composed of the column (11) and its ancillary devices, is different and is essentially replaced by two distillation columns arranged in series, an upper column D1 (21) and a lower column D2 (22), as described above. Thus, the entire process remains identical to that represented in Figure 1 and with the same reference numberings, up to the point where the gas stream G1 emerges via the line (10) no longer in the column (11) but in the column (21), in particular in the lower half, preferably in the bottom of the column (21). The process for withdrawing the gas stream G2, the condensation of G2 to form a condensate, the reflux of a portion of the condensate and recovery of the other additional portion of the condensate are identical to those illustrated in Figure 1 but are applied here no longer to the column (11), but to the column (21) and with the same reference numberings. A liquid transfer stream TL passes from the bottom of the column (21) via a line (23) to the top of the column (22). A gas transfer stream TG passes from the top of the column (22) via a line (25), via the line (10), to the bottom of the column (21). The column (22) is equipped at its base with a reboiler comprising a loop (12) and a heat exchanger (13), like those illustrated in Figure 1 at the base of the column (11). As in Figure 1, a line (14) withdraws the liquid stream L2 no longer from the bottom of the column (11) but from the bottom of the column (22). A liquid stream L3 is removed via a line (24) from the liquid transfer stream TL moving in the line (23) and is conveyed to the upper part, preferably to the top, of the column (7).

Figure 3 diagrammatically represents, by way of illustration, a process for the manufacture of ethylene oxide comprising the treatment process according to the invention and as illustrated in Figure 2 with the same reference numberings. The process for the manufacture of ethylene oxide comprises a reaction region (26) fed at the bottom, via a recycling loop (27), respectively with ethylene via a line (28), with molecular oxygen via a line (29), and with inert gas(es) via a line (30).

The gaseous reaction mixture described above exits at the top of the reaction region (26) via the recycling line (27) and subsequently enters an absorption column (31), preferably in the lower half of the column. A gas stream for recycling as described above exits at the top of the absorption column (31) via the recycling line (27). An essentially aqueous stream enters the absorption column (31) via a line (32), preferably in the upper half or at the top of the said column. A portion of the gas stream for recycling moving in the recycling line (27) at the outlet of the absorption column (31) is diverted into a line (33) equipped with a decarbonation region (34), as described above, so as to remove at least a portion of the carbon dioxide from the gas stream for recycling, before returning the gas stream for recycling, at least partially freed from carbon dioxide, via the line (33) to the recycling line (27) which subsequently returns to the bottom of the reaction region (26).

A dilute aqueous solution of impure ethylene oxide comprising the aldehyde impurities is withdrawn via a line (35) at the bottom of the absorption column (31) and is conveyed to a desorption column (36) as described above, preferably to the upper half or to the top of the latter column. Steam is introduced via a line (37) into the desorption column (36), preferably into the lower half or at the bottom of the said column. The liquid stream L1 withdrawn at the bottom of the distillation column (7) via the line (8) is conveyed to the desorption column (36), preferably to the lower half or to the bottom of the said column. An aqueous stream essentially free of ethylene oxide is withdrawn via a line (38) at the bottom of the desorption column (36), which stream can be partially discharged via a bleed line (39) and can be partially returned to the absorption column (31) via the line (32). The gaseous mixture of water and of impure ethylene oxide comprising the aldehyde impurities, formaldehyde and acetaldehyde, exits via the line (1) at the top of the desorption column (36) and is treated by the process of the invention, as illustrated in Figure 2.

In the continuation of the treatment according to the invention and as illustrated in Figure 3, all the operations are represented in an identical fashion to those illustrated in Figure 2, except for the fact that steam can be introduced as heat supply via a line (40) into the receiver (4) and that a gas stream comprising residual amounts of very volatile compound(s), such as carbon dioxide and optionally one or more inert gases, such as those mentioned above, can be entrained with the steam and ethylene oxide, can then be withdrawn at the top of the receiver (4) via the line (5) and can be conveyed to a column (41). The gas stream can encounter, in the column (41), countercurrentwise, water or an essentially aqueous stream introduced via a line (42), so that, on the one hand, the final traces of carbon dioxide gas and optionally of very volatile compound(s) and, on the other hand, the ethylene oxide entrained with the water in the form of a liquid stream are essentially separated. The latter liquid stream can be withdrawn at the bottom of the column (41) via a line (43), then be at least partially conveyed to the desorption column (36) via the line (8), and optionally discharged via a bleed (44). The additional portion not entrained in this liquid stream can be withdrawn at the top of the column (41) via a line (45) in the form of a gas stream comprising the residual carbon dioxide and optionally the very volatile compound or compounds. The latter gas stream can be at least partially conveyed via the line (45) to the decarbonation region (34), and can be recovered in the gas stream for recycling, and can optionally be discharged via a bleed (46).

The following example illustrates the present invention.

### Example

In a process for the manufacture of ethylene oxide by gas-phase catalytic oxidation of ethylene by oxygen, as represented diagrammatically in Figure 3, and which comprises a process for the treatment (according to the invention) of a mixture of water and impure ethylene oxide comprising the aldehyde impurities, formaldehyde and acetaldehyde, according to the diagram of Figure 2. This mixture arrives via a line (1) in the form of a gas stream at 89°C under an absolute pressure of 0.15 MPa and emerges in a condenser (2), so as to form an aqueous solution of impure ethylene oxide comprising the aldehyde impurities, formaldehyde and acetaldehyde. The aqueous solution comprises, for a mixture of 100 parts by weight of ethylene oxide and of water in a ratio by weight of ethylene oxide to water of 45/55, 25 ppm of formaldehyde, 85 ppm of acetaldehyde and 150 ppm of carbon dioxide gas. In a fractionation region Z1 composed of a distillation column (7) comprising 21 theoretical plates, the aqueous solution is introduced via a line (6) according to a flow rate of 26 t/h at a corresponding level between the 3rd and 7th theoretical plates, preferably at a level corresponding to the 6^{th} theoretical plate, counting from the bottom of the column. Steam is introduced according to a flow rate of 11 t/h via a line (9) in the bottom of the column (7), supplying the heat necessary to reach a column bottom temperature of 136°C and a column top temperature of 45°C, under an absolute pressure of 0.3 MPa. A liquid stream L1, comprising 99.3 % by weight of water, 0.07 % by weight of ethylene oxide, formaldehyde and acetaldehyde, is withdrawn at the bottom of the column (7) via a line (8) according to a flow rate of 18 t/h. A gas stream G1, comprising 99.9 % by weight of ethylene oxide, 60 ppm of water, formaldehyde and acetaldehyde, is withdrawn at the top of the column (7) via a line (10) according to a flow rate of 42 t/h.

A second fractionation region Z2 is composed of two distillation columns arranged in series: an upper distillation column D1 (21), comprising 66 theoretical plates, and a lower distillation column D2 (22), comprising 30 theoretical plates. The gas stream G1 feeds the bottom of the column (21) according to a flow rate of 42 t/h. The column (21) has a bottom temperature of 45°C and a top temperature of 40°C, under an absolute pressure of 0.3 MPa. A gas stream G2 is withdrawn at the top of the column (21) via a line (15) according to a flow rate of 76 t/h and is condensed and cooled in a condenser (16), so as to form a liquid stream which flows at 27°C via a line (17) out of the condenser (16). A portion of this liquid stream is refluxed via a line (18) according to a flow rate of 58 t/h to the top of the column (21). The additional non-refluxed portion is recovered via a line (19) according to a flow rate of 18 t/h and constitutes the purified ethylene oxide essentially free of water and of the aldehyde impurities, formaldehyde and acetaldehyde, resulting from the process of the invention. The purified ethylene oxide thus recovered comprises 3 ppm of formaldehyde, 4 ppm of acetaldehyde, 5 ppm of water and 4 ppm of carbon dioxide gas. At the bottom of the column (21), a liquid transfer stream TL flows via a line (23) and is introduced at the top of the column (22) according to a flow rate of 37.6 t/h. A portion of TL is removed from the line (23) to form a liquid stream L3, which is conveyed via a line (24) according to a flow rate of 23 t/h to the top of the distillation column (7), as reflux.

The column (22) is equipped at the bottom with a reboiler comprising a loop (12) and a heat exchanger (13) which makes it possible to maintain a bottom temperature of 47°C and a top temperature of 45°C, under an absolute pressure of 0.3 MPa. A liquid stream L2, comprising 90 % by weight of ethylene oxide, 2.5 % by weight of water and some acetaldehyde, is withdrawn at the bottom of the column (22) via a line (14) according to a flow rate of 1 t/h. A gas transfer stream TG is withdrawn at the top of the column (22) via a line (25) according to a flow rate of 36.6 t/h and is conveyed to the bottom of the column (21) via the line (10).

## Claims

1. Process for treating a mixture of impure ethylene oxide and of water in a ratio by weight of ethylene oxide to water of 0.1/1 to 4/1, comprising aldehyde impurities, formaldehyde and acetaldehyde, in order to obtain a purified ethylene oxide essentially free of water and of the aldehyde impurities, which process is **characterized in that**:
(1) the mixture is conveyed to a first fractionation region Z1, while a liquid stream L1 comprising at least 98 % by weight of water and a portion of the aldehyde impurities is withdrawn at the bottom of Z1, and a gas stream G1 comprising more than 99 % by weight of ethylene oxide with residual amounts of water and of the aldehyde impurities is withdrawn at the top of Z1,
(2) the gas stream G1 is conveyed to a second fractionation region Z2, while a liquid stream L2 comprising a mixture of ethylene oxide, of water and of the aldehyde impurity(ies) is withdrawn at the bottom of Z2, a liquid stream L3 is withdrawn as a side stream from the region Z2 and is conveyed to the region Z1, and a gas stream G2 comprising the purified ethylene oxide essentially free of water and of the aldehyde impurities is withdrawn at the top of Z2.

2. Process according to Claim 1, **characterized in that** the mixture of impure ethylene oxide and of water is a gas mixture which, prior to being sent to the region Z1, is condensed, so as to form an aqueous solution of impure ethylene oxide.

3. Process according to Claim 1 or 2, **characterized in that** the amount of the aldehyde impurities, formaldehyde and acetaldehyde, in the mixture of impure ethylene oxide and of water conveyed to the region Z1 is from 50 to 2 000 parts per million by weight (ppm).

4. Process according to any one of Claims 1 to 3, **characterized in that** the region Z1 comprises at least one distillation column having from 10 to 30 theoretical plates.

5. Process according to any one of Claims 1 to 4, **characterized in that** the region Z1 is fed with a mixture of impure ethylene oxide and of water at a level situated between a tenth and a half of the number of theoretical plates of Z1, counting from the bottom of Z1.

6. Process according to any one of Claims 1 to 5, **characterized in that** heat is supplied to the region Z1 by virtue of a reboiler positioned in the bottom of Z1 or, preferably, by virtue of the introduction of steam into the bottom of Z1.

7. Process according to any one of Claims 1 to 6, **characterized in that** the region Z1 operates with a bottom temperature of 100 to 160°C and a top temperature of 30 to 60°C, under an absolute pressure of 0.1 to 1 MPa.

8. Process according to any one of Claims 1 to 7, **characterized in that** the streams G1 and L1 are withdrawn from the region Z1 in a ratio by weight of G1 to L1 of 1/1 to 5/1.

9. Process according to any one of Claims 1 to 8, **characterized in that** the liquid stream L1 comprises less than 0.5 % by weight of ethylene oxide.

10. Process according to any one of Claims 1 to 9, **characterized in that** the liquid stream L1 comprises from 60 to 99 % by weight of the amount of formaldehyde present in the mixture of impure ethylene oxide and of water conveyed to the region Z1.

11. Process according to any one of Claims 1 to 10, **characterized in that** the region Z2 comprises at least one distillation column having from 50 to 120 theoretical plates.

12. Process according to any one of Claims 1 to 11, **characterized in that** heat is supplied to the region Z2 by virtue of the introduction of steam into the bottom of Z2 or, preferably, by virtue of a reboiler positioned in the bottom of Z2.

13. Process according to any one of Claims 1 to 12, **characterized in that** the region Z2 operates with a bottom temperature of 35 to 65°C and a top temperature of 30 to 50°C, under an absolute pressure different from or, preferably, substantially identical to that existing in the region Z1, in particular ranging from 0.1 to 1 MPa.

14. Process according to any one of Claims 1 to 13, **characterized in that** the gas stream G2 withdrawn at the top of Z2 is condensed, so as to form a condensate, a portion of the resulting condensate being returned as reflux to the top of the region Z2 and the additional non-refluxed portion being recovered in the form of a liquid stream of the purified ethylene oxide essentially free of water and of the aldehyde impurities, preferably in a ratio by weight of the refluxed part to the additional non-refluxed part of 1/1 to 10/1.

15. Process according to any one of Claims 1 to 14, **characterized in that** the gas stream G1 feeds the region Z2 at a level situated between a quarter and a half of the number of theoretical plates of Z2, counting from the bottom of Z2.

16. Process according to any one of Claims 1 to 15, **characterized in that** the streams G2 and L2 are withdrawn from the region Z2 in a ratio by weight of G2 to L2 of 30/1 to 150/1.

17. Process according to any one of Claims 1 to 16, **characterized in that**, per 100 parts by weight of the liquid stream L2, from 80 to 95 parts by weight of ethylene oxide, from 1 to 5 parts by weight of water and from 1 to 5 parts by weight of the aldehyde impurity(ies), in particular of acetaldehyde, are recorded.

18. Process according to any one of Claims 1 to 17, **characterized in that** the purified ethylene oxide essentially free of water and of the aldehyde impurities resulting from the gas stream G2 comprises from 1 to 50 ppm of water and from 1 to less than 15 ppm of the aldehyde impurities.

19. Process according to Claim 18, **characterized in that** the purified ethylene oxide essentially free of water and of the aldehyde impurities comprises less than 5 ppm of formaldehyde.

20. Process according to any one of Claims 1 to 19, **characterized in that** the liquid stream L3 is withdrawn from Z2 at the same level as or below the point for feeding Z2 with gas stream G1.

21. Process according to any one of Claims 1 to 20, **characterized in that** the liquid stream L3 is withdrawn from Z2 at a level situated between a quarter and a half of the number of theoretical plates, counting from the bottom of Z2.

22. Process according to any one of Claims 1 to 21, **characterized in that** the liquid stream L3 is conveyed to a point situated in the upper half of the region Z1, preferably to the top of Z1.

23. Process according to any one of Claims 1 to 22, **characterized in that** the streams G1 and L3 are employed in a ratio by weight of G1 to L3 of 0.5/1 to 5/1.

24. Process according to any one of Claims 1 to 23, **characterized in that** the streams G2 and L3 are withdrawn from the region Z2 in a ratio by weight of G2 to L3 of 1/1 to 20/1.

25. Process according to any one of Claims 1 to 24, **characterized in that** the fractionation region Z2 comprises two distillation columns arranged in series, an upper column D1 and a lower column D2, so that the gas stream G1 emerges in the lower half of D1, preferably in the bottom of D1, so that the gas stream G2 exits via the top of D1, so that the bottom of D1 communicates via a liquid transfer stream TL with the top of D2, so that the top of D2 communicates via a gas transfer stream TG with the bottom of D1, and so that the liquid stream L2 exits via the bottom of D2.

26. Process according to Claim 25, **characterized in that** the gas transfer stream TG emerges in the gas stream G1, before being introduced into the lower half of D1 or, preferably, into the bottom of D1.

27. Process according to Claim 25 or 26, **characterized in that** the liquid stream L3 is removed from the liquid transfer stream TL.

28. Process for the manufacture of a purified ethylene oxide essentially free of water and of aldehyde impurities, formaldehyde and acetaldehyde, comprising:
(a) synthesis of ethylene oxide in a reaction region by a gas-phase catalytic reaction between ethylene and molecular oxygen, so as to form a gaseous reaction mixture comprising the ethylene oxide formed, the aldehyde impurities, unreacted reactants and possibly inert gases,
(b) absorption with water by bringing the gaseous reaction mixture obtained in (a) into contact with an essentially aqueous stream, so as to form a dilute aqueous solution of impure ethylene oxide comprising the aldehyde impurities, after separation of a gas stream for recycling essentially comprising the unreacted reactants and possibly the inert gases, which gas stream is at least partially returned, directly or indirectly, to the reaction region, and
(c) desorption of the ethylene oxide by entrainment of the dilute aqueous solution obtained in (b) with steam in a column, so as to form, at the column bottom, an aqueous stream essentially free of ethylene oxide and, at the top, a mixture of impure ethylene oxide and of water in a ratio by weight of ethylene oxide to water of 0.1/1 to 4/1, comprising the aldehyde impurities,
which process is **characterized in that** the mixture of impure ethylene oxide and of water obtained in (c) is subjected to the treatment according to any one of Claims 1 to 27.

29. Process according to Claim 28, **characterized in that** the gas stream for recycling obtained in (b) is subjected to decarbonation, before it is returned to the reaction region.

## Patentansprüche

1. Verfahren zum Behandeln eines Gemisches von unreinem Ethylenoxid und von Wasser in einem Gewichtsverhältnis, von Ethylenoxid zu Wasser von 0,1:1 bis 4:1, umfassend Aldehydverunreinigungen, Formaldehyd und Acetaldehyd, um ein gereinigtes Ethylenoxid, das im Wesentlichen frei von Wasser und den Aldehydverunreinigungen ist, zu erhalten, wobei das Verfahren **dadurch gekennzeichnet ist, dass**:
(1) das Gemisch zu einem ersten Fraktionierungsbereich Z1 geleitet wird, während ein Flüssigkeitsstrom L1, umfassend mindestens 98 Gewichtsprozent Wasser und einen Teil der Aldehydverunreinigungen, am Sumpf von Z1 abgezogen wird, und ein Gasstrom G1, umfassend mehr als 99 Gewichtsprozent Ethylenoxid, mit restlichen Mengen an Wasser und den Aldehydverunreinigungen von dem Kopf von Z1 abgezogen wird,
(2) der Gasstrom G1 zu einem zweiten Fraktionierungsbereich Z2 geleitet wird, während ein Flüssigkeitsstrom L2, umfassend ein Gemisch von Ethylenoxid, von Wasser und von der/den Aldehydverunreinigung(en), am Sumpf von Z2 abgezogen wird, ein Flüssigkeitsstrom L3 als ein Nebenstrom von dem Bereich Z2 abgezogen wird und zu dem Bereich Z1 geleitet wird, und ein Gasstrom G2, umfassend das gereinigte Ethylenoxid, das im Wesentlichen frei von Wasser und den Aldehydverunreinigungen ist, am Kopf von Z2 abgezogen wird.

2. Verfahren nach Anspruch 1, **dadurch gekennzeichnet, dass** das Gemisch von unreinem Ethylenoxid und von Wasser ein Gasgemisch ist, das, bevor es zu dem Bereich Z1 geschickt wird, kondensiert wird, um eine wässrige Lösung von unreinem Ethylenoxid zu bilden.

3. Verfahren nach Anspruch 1 oder 2, **dadurch gekennzeichnet, dass** die Menge der Aldehydverunreinigungen, Formaldehyd und Acetaldehyd in dem Gemisch von unreinem Ethylenoxid und von Wasser, geleitet zu dem Bereich Z1, 50 bis 2 000 Gewichtsteile pro Millionen (ppm) ist.

4. Verfahren nach einem der Ansprüche 1 bis 3, **dadurch gekennzeichnet, dass** der Bereich Z1 mindestens eine Destillationskolonne mit 10 bis 30 theoretischen Böden umfasst.

5. Verfahren nach einem der Ansprüche 1 bis 4, **dadurch gekennzeichnet, dass** der Bereich Z1 mit einem Gemisch von unreinem Ethylenoxid und von Wasser bei einem Niveau, angeordnet zwischen einem Zehntel und einer Hälfte der Anzahl an theoretischen Böden Z1, gerechnet vom Sumpf von Z1 an, beschickt wird.

6. Verfahren nach einem der Ansprüche 1 bis 5, **dadurch gekennzeichnet, dass** Wärme dem Bereich Z1 auf Grund eines Reboilers, der zwischen dem Sumpf Z1 angeordnet ist, oder vorzugsweise auf Grund der Einführung von Dampf in den Sumpf von Z1 zugeführt wird.

7. Verfahren nach einem der Ansprüche 1 bis 6, **dadurch gekennzeichnet, dass** der Bereich Z1 mit einer Sumpftemperatur von 100 bis 160°C und einer Kopftemperatur von 30 bis 60°C unter einem Absolutdruck von 0,1 bis 1 MPa betrieben wird.

8. Verfahren nach einem der Ansprüche 1 bis 7, **dadurch gekennzeichnet, dass** die Ströme G1 und L1 von dem Bereich Z1 in einem Gewichtsverhältnis von G1:L1 von 1:1 bis 5:1 abgezogen werden.

9. Verfahren nach einem der Ansprüche 1 bis 8, **dadurch gekennzeichnet, dass** der Flüssigkeitsstrom L1 weniger als 0,5 Gewichtsprozent Ethylenoxid umfasst.

10. Verfahren nach einem der Ansprüche 1 bis 9, **dadurch gekennzeichnet, dass** der Flüssigkeitsstrom L1 60 bis 99 Gewichtsprozent der Menge an vorliegendem Formaldehyd umfasst, die in dem Gemisch von unreinem Ethylenoxid und von Wasser, geleitet zu dem Bereich Z1, vorliegt.

11. Verfahren nach einem der Ansprüche 1 bis 10, **dadurch gekennzeichnet, dass** der Bereich Z2 mindestens eine Destillationskolonne mit 50 bis 120 theoretischen Böden umfasst.

12. Verfahren nach einem der Ansprüche 1 bis 11, **dadurch gekennzeichnet, dass** Wärme zu dem Bereich Z2 auf Grund der Einführung von Dampf in den Sumpf von Z2, oder vorzugsweise auf Grund eines Reboilers, der in dem Sumpf von Z2 angeordnet ist, zugeführt wird.

13. Verfahren nach einem der Ansprüche 1 bis 12, **dadurch gekennzeichnet, dass** der Bereich Z2 mit einer Sumpftemperatur von 35 bis 65°C und einer Kopftemperatur von 30 bis 50°C unter einem Absolutdruck, der sich unterscheidet von, oder vorzugsweise im Wesentlichen identisch ist mit jenem, der in dem Bereich Z1 vorliegt, insbesondere im Bereich von 0,1 bis 1 MPa, betrieben wird.

14. Verfahren nach einem der Ansprüche 1 bis 13, **dadurch gekennzeichnet, dass** der Gasstrom G2, der von dem Kopf von Z2 abgezogen wird, kondensiert wird, sodass ein Kondensat gebildet wird, wobei ein Teil des erhaltenen Kondensats als Rückfluss zu dem Kopf des Bereichs Z2 zurückgeführt wird, und der zusätzliche, nicht unter Rückfluss erhitzte Teil in Form eines Flüssigkeitsstroms von dem gereinigten Ethylenoxid, das im Wesentlichen frei von Wasser und von den Aldehydverunreinigungen ist, vorzugsweise in einem Gewichtsverhältnis auf das von dem unter Rückfluss erhitzten Teil zu dem zusätzlichen, nicht unter Rückfluss erhitzten Teil von 1:1 bis 10:1 wieder gewonnen wird.

15. Verfahren nach einem der Ansprüche 1 bis 14, **dadurch gekennzeichnet, dass** der Gasstrom G1 den Bereich Z2 bei einem Niveau, angeordnet zwischen einem Viertel und einer Hälfte der Anzahl an theoretischen Böden von Z2, gerechnet vom Sumpf von Z2 an, versorgt.

16. Verfahren nach einem der Ansprüche 1 bis 15, **dadurch gekennzeichnet, dass** die Ströme G2 und L2 von dem Bereich Z2 in einem Gewichtsverhältnis von G2:L2 von 30:1 bis 150:1 abgezogen wird.

17. Verfahren nach einem der Ansprüche 1 bis 16, **dadurch gekennzeichnet, dass** pro 100 Gewichtsteile des Flüssigkeitsstroms L2 80 bis 95 Gewichtsteile Ethylenoxid, 1 bis 5 Gewichtsteile Wasser und 1 bis 5 Gewichtsteile von der/den Aldehydverunreinigung(en), insbesondere Acetaldehyd, aufgezeichnet werden.

18. Verfahren nach einem der Ansprüche 1 bis 17, **dadurch gekennzeichnet, dass** das gereinigte Ethylenoxid, das im Wesentlichen frei von Wasser und den Aldehydverunreinigungen ist, das aus dem Gasstrom G2 stammt, 1 bis 50 ppm Wasser und 1 bis weniger als 15 ppm Aldehydverunreinigungen umfasst.

19. Verfahren nach Anspruch 18, **dadurch gekennzeichnet, dass** das gereinigte Ethylenoxid, das im Wesentlichen frei von Wasser und Aldehydverunreinigungen ist, weniger als 5 ppm Formaldehyd umfasst.

20. Verfahren nach einem der Ansprüche 1 bis 19, **dadurch gekennzeichnet, dass** der Flüssigkeitsstrom L3 von Z2 bei dem gleichen Niveau wie oder unterhalb des Punktes zur Zuführung von Z2 mit dem Gasstrom G1 abgezogen wird.

21. Verfahren nach einem der Ansprüche 1 bis 20, **dadurch gekennzeichnet, dass** der Flüssigkeitsstrom L3 von Z2 bei einem Niveau, angeordnet zwischen einem Viertel und einer Hälfte der Anzahl an theoretischen Böden, gerechnet vom Sumpf von Z2 an, abgezogen wird.

22. Verfahren nach einem der Ansprüche 1 bis 21, **dadurch gekennzeichnet, dass** der Flüssigkeitsstrom L3 zu einem Punkt, angeordnet in der oberen Hälfte des Bereichs Z1, vorzugsweise zu dem Kopf von Z1 transportiert wird.

23. Verfahren nach einem der Ansprüche 1 bis 22, **dadurch gekennzeichnet, dass** die Ströme G1 und L3 in einem Gewichtsverhältnis von G1:L3 von 0,5:1 bis 5:1 angewendet werden.

24. Verfahren nach einem der Ansprüche 1 bis 23, **dadurch gekennzeichnet, dass** die Ströme G2 und L3 aus dem Bereich Z2 in einem Gewichtsverhältnis von G2:L3 von 1:1 bis 20:1 abgezogen werden.

25. Verfahren nach einem der Ansprüche 1 bis 24, **dadurch gekennzeichnet, dass** der Fraktionierungsbereich Z2 zwei in Reihe angeordnete Destillationskolonnen, eine obere Kolonne D1 und eine untere Kolonne D2, umfasst, sodass der Gasstrom G1 in die untere Hälfte von D1, vorzugsweise in den Sumpf von D1, mündet, sodass der Gasstrom G2 über dem Kopf von D1 austritt, sodass der Sumpf von D1 über einen Flüssigkeitstransferstrom TL mit dem Kopf von D2 kommuniziert, sodass der Kopf von D2 über einen Gastransferstrom TG mit dem Sumpf von D1 kommuniziert, und sodass der Flüssigkeitsstrom L2 über den Sumpf von D2 austritt.

26. Verfahren nach Anspruch 25, **dadurch gekennzeichnet, dass** der Gastransferstrom TG in dem Gasstrom G1 mündet, bevor er in die untere Hälfte von D1 oder vorzugsweise in den Sumpf von D1 eingeführt wird.

27. Verfahren nach Anspruch 25 oder 26, **dadurch gekennzeichnet, dass** der Flüssigkeitsstrom L3 aus dem Flüssigkeitstransferstrom TL entfernt wird.

28. Verfahren zur Herstellung von einem gereinigten Ethylenoxid, das im Wesentlichen frei von Wasser und Aldehydverunreinigungen, Formaldehyd und Acetaldehyd ist, umfassend:
(a) Synthese von Ethylenoxid in einem Reaktionsbereich durch eine katalytische Gasphasen-Reaktion zwischen Ethylen und molekularem Sauerstoff, sodass ein gasförmiges Reaktionsgemisch gebildet wird, umfassend das gebildete Ethylenoxid, die Aldehydverunreinigungen, nicht umgesetzte Reaktanten und möglicherweise Inertgase,
(b) Absorption mit Wasser durch Bringen des in (a) erhaltenen, gasförmigen Reaktionsgemisches in Kontakt mit einem im Wesentlichen wässrigen Strom, sodass eine verdünnte wässrige Lösung von unreinem, die Aldehydverunreinigungen umfassendem Ethylenoxid gebildet wird, nach Abtrennung eines Gasstroms zum Zurückführen, im Wesentlichen umfassend die nicht umgesetzten Reaktanten und möglicherweise die Inertgase, wobei der Gasstrom mindestens teilweise direkt oder indirekt zu dem Reaktionsbereich zurückgeführt wird, und
(c) Desorption von dem Ethylenoxid durch Mitreißen von der in (b) erhaltenen, verdünnten wässrigen Lösung mit Dampf in einer Kolonne, sodass an dem Kolonnensumpf einen wässrigen Strom, der im Wesentlichen frei von Ethylenoxid ist, und am Kopf ein Gemisch von unreinem Ethylenoxid und von Wasser in einem Gewichtsverhältnis von Ethylenoxid zu Wasser von 0,1:1 bis 4:1, umfassend die Aldehydverunreinigungen, gebildet wird,
wobei das Verfahren **dadurch gekennzeichnet ist, dass** das Gemisch von unreinem Ethylenoxid und von in (c) erhaltenem Wasser der Behandlung nach einem der Ansprüche 1 bis 27 unterzogen wird.

29. Verfahren nach Anspruch 28, **dadurch gekennzeichnet, dass** der in (b) erhaltene Gasstrom zum Zurückführen Decarbonisierung unterzogen wird, bevor er zu dem Reaktionsbereich zurückgeführt wird.

## Revendications

1. Procédé de traitement d'un mélange d'oxyde d'éthylène impur et d'eau dans un rapport en poids d'oxyde d'éthylène sur l'eau de 0,1/1 à 4/1, comprenant des impuretés de type aldéhyde, de formaldéhyde et d'acétaldéhyde, afin d'obtenir un oxyde d'éthylène purifié essentiellement sans eau et sans impuretés de type aldéhyde, lequel procédé est **caractérisé en ce que** :
(1) le mélange est déplacé dans une première zone de fractionnement Z1, alors qu'un flux liquide L1 comprenant au moins 98 % en poids d'eau et une partie des impuretés de type aldéhyde est retiré du fond de Z1, et un flux de gaz G1 comprenant plus de 99 % en poids d'oxyde d'éthylène avec des quantités résiduelles d'eau et des impuretés de type aldéhyde est retiré au niveau de la partie supérieure de Z1,
(2) le flux de gaz G1 est déplacé dans une seconde zone de fractionnement Z2, alors qu'un flux liquide L2 comprenant un mélange d'oxyde d'éthylène, d'eau et d'une ou plusieurs impuretés de type aldéhyde est retiré du fond de Z2, un flux liquide L3 est retiré en tant que flux secondaire de la zone Z2 et est transféré vers la zone Z1, et un flux de gaz G2 comprenant l'oxyde d'éthylène purifié essentiellement sans eau et sans impureté de type aldéhyde est retiré au niveau de la partie supérieure de Z2.

2. Procédé selon la revendication 1, **caractérisé en ce que** le mélange d'oxyde d'éthylène impur et d'eau est un mélange de gaz qui, avant d'être envoyé dans la zone Z1, est condensé, afin de former une solution aqueuse d'oxyde d'éthylène impur.

3. Procédé selon la revendication 1 ou 2, **caractérisé en ce que** la quantité des impuretés de type aldéhyde, de formaldéhyde et d'acétaldéhyde, dans le mélange d'oxyde d'éthylène impur et d'eau transféré vers la zone Z1 est de 50 à 2000 parties par million en poids (ppm).

4. Procédé selon l'une quelconque des revendications 1 à 3, **caractérisé en ce que** la zone Z1 comprend au moins une colonne de distillation ayant de 10 à 30 plateaux théoriques.

5. Procédé selon l'une quelconque des revendications 1 à 4, **caractérisé en ce que** la zone Z1 est alimentée d'un mélange d'oxyde d'éthylène impur et d'eau à un niveau situé entre un dixième et la moitié du nombre de plateaux théoriques de Z1, en comptant depuis le fond de Z1.

6. Procédé selon l'une quelconque des revendications 1 à 5, **caractérisé en ce que** la chaleur est fournie à la zone Z1 en vertu d'un rebouilleur placé au fond de Z1 ou, de préférence, en vertu de l'introduction de vapeur dans le fond de Z1.

7. Procédé selon l'une quelconque des revendications 1 à 6, **caractérisé en ce que** la zone Z1 fonctionne avec une température du fond allant de 100 à 160°C et une température de la partie supérieure allant de 30 à 60°C, sous une pression absolue de 0,1 à 1 MPa.

8. Procédé selon l'une quelconque des revendications 1 à 7, **caractérisé en ce que** les flux G1 et L1 sont retirés de la zone Z1 dans un rapport en poids en poids de G1 sur L1 de 1/1 à 5/1.

9. Procédé selon l'une quelconque des revendications 1 à 8, **caractérisé en ce que** le flux liquide L1 comprend moins de 0,5 % en poids d'oxyde d'éthylène.

10. Procédé selon l'une quelconque des revendications 1 à 9, **caractérisé en ce que** le flux liquide L1 comprend de 60 à 99 % en poids de la quantité de formaldéhyde présente dans le mélange d'oxyde d'éthylène impur et d'eau déplacé dans la zone Z1.

11. Procédé selon l'une quelconque des revendications 1 à 10, **caractérisé en ce que** la zone Z2 comprend au moins une colonne de distillation ayant de 50 à 120 plateaux théoriques.

12. Procédé selon l'une quelconque des revendications 1 à 11, **caractérisé en ce que** la chaleur est fournie à la zone Z2 en vertu de l'introduction de la vapeur dans le fond de Z2 ou, de préférence, en vertu d'un rebouilleur placé dans le fond de Z2.

13. Procédé selon l'une quelconque des revendications 1 à 12, **caractérisé en ce que** la zone Z2 fonctionne avec une température du fond allant de 35 à 65°C et une température de la partie supérieure allant de 30 à 50°C, sous une pression absolue différente ou, de préférence, essentiellement identique à celle existant dans la zone Z1, en particulier comprise dans la gamme allant de 0,1 à 1 MPa.

14. Procédé selon l'une quelconque des revendications 1 à 13, **caractérisé en ce que** le flux de gaz G2 retiré au niveau de la partie supérieure de Z2 est condensé, de façon à former un condensat, une partie du condensat résultant retournant sous la forme de reflux au niveau de la partie supérieure de la zone Z2 et la partie supplémentaire qui n'est pas chauffée au reflux étant récupérée sous forme d'un flux liquide de l'oxyde d'éthylène purifié essentiellement sans eau et sans impureté de type aldéhyde, de préférence dans un rapport en poids de la partie chauffée au reflux sur la partie supplémentaire qui n'a pas été chauffée au reflux de 1/1 à 10/1.

15. Procédé selon l'une quelconque des revendications 1 à 14, **caractérisé en ce que** le flux de gaz G1 alimente la zone Z2 à un niveau situé entre un quart et la moitié du nombre de plateaux théoriques de Z2, en comptant à partir du fond de Z2.

16. Procédé selon l'une quelconque des revendications 1 à 15, **caractérisé en ce que** les flux G2 et L2 sont retirés de la zone Z2 dans un rapport en poids de G2 sur L2 de 30/1 à 150/1.

17. Procédé selon l'une quelconque des revendications 1 à 16, **caractérisé en ce que**, pour 100 parties en poids du flux liquide L2, de 80 à 95 parties en poids d'oxyde d'éthylène, de 1 à 5 parties en poids d'eau et de 1 à 5 parties en poids d'une ou plusieurs impuretés de type aldéhyde, en particulier d'acétaldéhyde, sont enregistrées.

18. Procédé selon l'une quelconque des revendications 1 à 17, **caractérisé en ce que** l'oxyde d'éthylène purifié essentiellement sans eau et sans impureté de type aldéhyde résultant du flux de gaz G2 comprend de 1 à 50 ppm d'eau et de 1 à moins de 15 ppm d'impuretés de type aldéhyde.

19. Procédé selon la revendication 18, **caractérisé en ce que** l'oxyde d'éthylène purifié essentiellement sans eau et sans impureté de type aldéhyde comprend moins de 5 ppm de formaldéhyde.

20. Procédé selon l'une quelconque des revendications 1 à 19, **caractérisé en ce que** le flux liquide L3 est retiré de Z2 au même niveau ou au-dessous du point d'alimentation de Z2 avec le flux de gaz G1.

21. Procédé selon l'une quelconque des revendications 1 à 20, **caractérisé en ce que** le flux liquide L3 est retiré de Z2 à un niveau situé entre un quart et la moitié du nombre de plateaux théoriques, en comptant à partir du fond de Z2.

22. Procédé selon l'une quelconque des revendications 1 à 21, **caractérisé en ce que** le flux liquide L3 est transféré vers un point situé dans la moitié supérieure de la zone Z1, de préférence sur la partie supérieure de Z1.

23. Procédé selon l'une quelconque des revendications 1 à 22, **caractérisé en ce que** les flux G1 et L3 sont utilisés dans un rapport en poids de G1 sur L3 de 0,5/1 à 5/1.

24. Procédé selon l'une quelconque des revendications 1 à 23, **caractérisé en ce que** les flux G2 et L3 sont retirés de la zone Z2 dans un rapport en poids de G2 sur L3 de 1/1 à 20/1.

25. Procédé selon l'une quelconque des revendications 1 à 24, **caractérisé en ce que** la zone de fractionnement Z2 comprend deux colonnes de distillation disposées en série, une colonne supérieure D1 et une colonne inférieure D2, de sorte que le flux de gaz G1 émerge dans la moitié inférieure de D1, de préférence au fond de D1, de sorte que le flux de gaz G2 sorte par l'intermédiaire de la partie supérieure de D1, de sorte que le fond de D1 communique par l'intermédiaire d'un flux de transfert liquide TL avec la partie supérieure de D2, de sorte que la partie supérieure de D2 communique par l'intermédiaire d'un flux de transfert de gaz TG avec le fond de D1, et de sorte que le flux liquide L2 sorte par l'intermédiaire du fond de D2.

26. Procédé selon la revendication 25, **caractérisé en ce que** le flux de transfert de gaz TG émerge dans le flux de gaz G1, avant d'être introduit dans la moitié inférieure de D1 ou, de préférence, dans le fond de D1.

27. Procédé selon la revendication 25 ou 26, **caractérisé en ce que** le flux liquide L3 est retiré du flux de transfert liquide TL.

28. Procédé destiné à la fabrication d'un oxyde d'éthylène purifié essentiellement sans eau et sans impureté de type aldéhyde, formaldéhyde et acétaldéhyde, comprenant :
(a) la synthèse d'oxyde d'éthylène dans une zone de réaction par une réaction catalytique en phase gazeuse entre l'éthylène et de l'oxygène moléculaire, de façon à former un mélange réactionnel gazeux comprenant l'oxyde d'éthylène formé, les impuretés de type aldéhyde, des réactifs qui n'ont pas réagi et probablement des gaz inertes,
(b) l'absorption dans de l'eau en mettant le mélange réactionnel gazeux obtenu en (a) en contact avec un flux essentiellement aqueux, de façon à former une solution aqueuse diluée d'oxyde d'éthylène impur comprenant les impuretés de type aldéhyde, après la séparation d'un flux de gaz destiné au recyclage comprenant essentiellement des réactifs qui n'ont pas réagi et probablement les gaz inertes, lequel flux de gaz est au moins partiellement renvoyé, directement ou indirectement, dans la zone de réaction, et
(c) la désorption de l'oxyde d'éthylène par l'entraînement de la solution aqueuse diluée obtenu en (b) avec de la vapeur dans une colonne, de façon à former, au fond de la colonne, un flux aqueux essentiellement sans oxyde d'éthylène et, au niveau de la partie supérieure, un mélange d'oxyde d'éthylène impur et d'eau dans un rapport en poids d'oxyde d'éthylène sur l'eau de 0,1/1 à 4/1, comprenant les impuretés de type aldéhyde,
lequel procédé est **caractérisé en ce que** le mélange d'oxyde d'éthylène impur et d'eau obtenu en (c) est soumis au traitement selon l'une quelconque des revendications 1 à 27.

29. Procédé selon la revendication 28, **caractérisé en ce que** le flux de gaz destiné au recyclage obtenu en (b) est soumis à la décarbonation, avant qu'il soit renvoyé dans la zone de réaction.
